(19) Europäisches Patentamt
European Patent Office
Office européen des brevets

(11) EP 3 045 519 A1

(12) EUROPEAN PATENT APPLICATION
published in accordance with Art. 153(4) EPC

(43) Date of publication:
20.07.2016 Bulletin 2016/29

(21) Application number: 13893185.2

(22) Date of filing: 09.09.2013

(51) Int Cl.:
*C12M 1/00* (2006.01) *C12N 5/071* (2010.01)

(86) International application number:
PCT/JP2013/074186

(87) International publication number:
WO 2015/033457 (12.03.2015 Gazette 2015/10)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Hitachi, Ltd.
Tokyo 100-8280 (JP)

(72) Inventors:
- TADA, Yasuhiko
Tokyo 100-8280 (JP)
- SHIBUYA, Keisuke
Tokyo 100-8280 (JP)
- SUGITA, Yui
Tokyo 100-8280 (JP)

(74) Representative: MERH-IP Matias Erny Reichl Hoffmann
Patentanwälte PartG mbB
Paul-Heyse-Strasse 29
80336 München (DE)

# (54) CELL SEPARATION AND COLLECTION MEMBRANE, AND CULTURING SHEET, CULTURING DEVICE, AND CELL SEPARATION AND COLLECTION METHOD USING SAME

(57) Provided are a cell separation and collection membrane capable of suppressing non-specific adsorption of cells while specifically adsorbing target cells; a culturing sheet and a culturing apparatus which use the membrane; and a cell separation and collection method. The cell separation and collection membrane included in the culturing sheet comprises: a support; a polymer site fixed to the support and having a changeable structure in accordance with a temperature; a cell adsorbing site bound to the polymer site, exposed relative to an outer surface of the support, and specifically adsorbing a target cell when the cell adsorbing site is brought into contact with a treatment solution containing the target cells; and a hydrophilic site exposed relative to the outer surface and to be brought into contact with the treatment solution.

FIG.2A

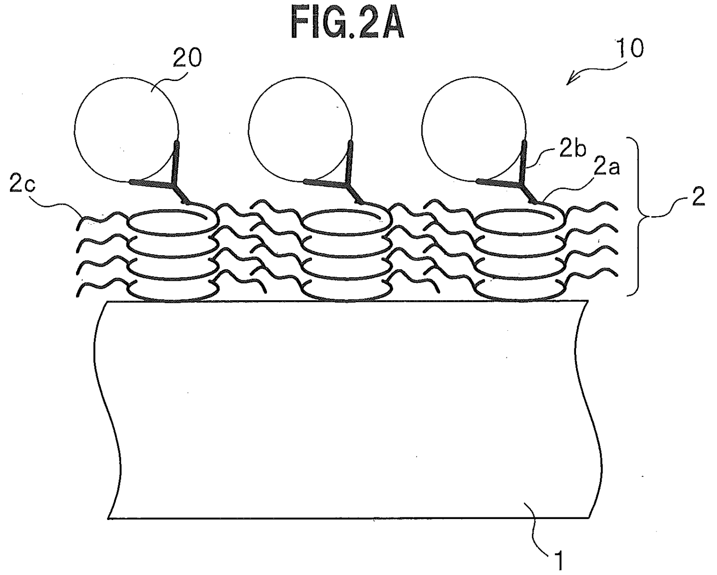


EP 3 045 519 A1

FIG.2B

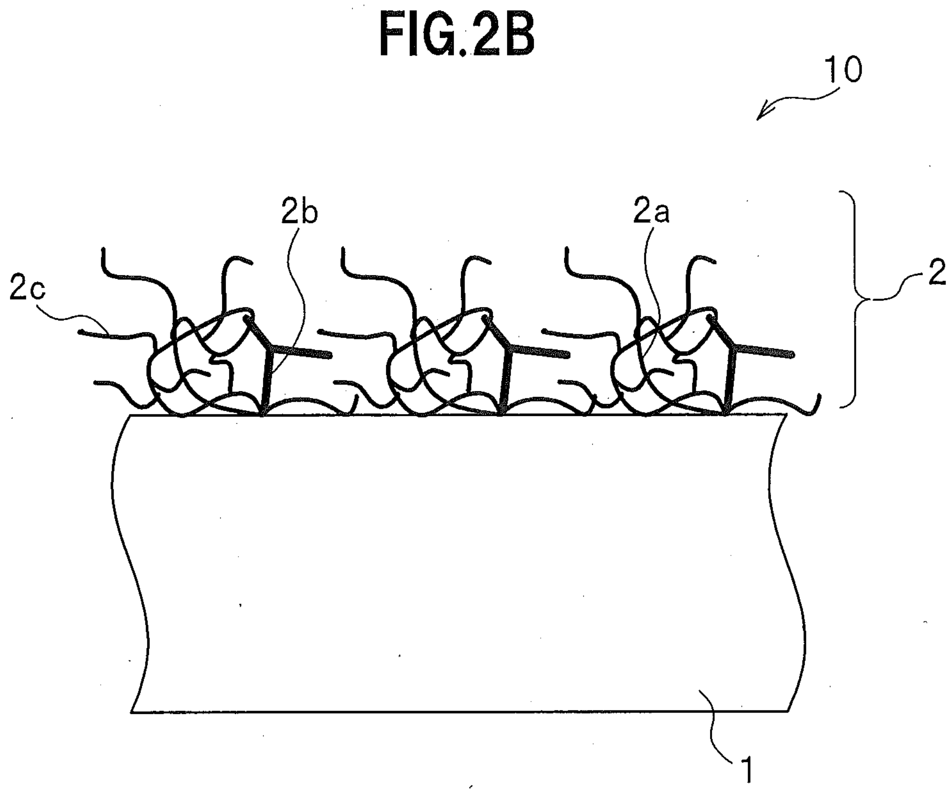

## Description

### TECHNICAL FIELD

The present invention relates to a cell separation and collection membrane, a culturing sheet and a culturing apparatus which use the membrane, and a cell separation and collection method.

### BACKGROUND ART

Recently, the need for techniques for specifically (or selectively) separating and collecting cells has been increasing in the fields of medicine, biology, immunology, and the like. For example, in the field of regenerative medicine, target cells are specifically collected on a substrate of glass, polymer, or the like. Then, the collected target cells are cultured on the substrate, and subsequently the target cells are released and separated from the substrate. When the cultured target cells are released from the substrate, trypsin (i.e., a protease) is used in many cases.

However, the process of separating and collecting target cells is complicated. Hence, separating and collecting steps are time-consuming. Particularly, selecting an effective bacterium in soil, water, or the like sometimes requires a considerably long time because the selection includes screening, and culturing the effective bacterium obtained by the screening. Accordingly, there has been a demand for a technique for separating and collecting cells in a short time.

Further, when cells cultured on a substrate are released from the substrate by using trypsin or the like, trypsin or the like may somewhat influence the cultured cells. Accordingly, there is a demand for a technique for separating and collecting cells from a substrate without using an enzyme, agent, or the like as much as possible.

In view of those circumstances, a technique described in Patent Literature 1 is known. Patent Literature 1 describes a cell support including a polymer film made of a temperature responsive polymer showing an upper critical dissolution temperature or a lower critical dissolution temperature of 0 to 80°C in water, and configured to retain an antibody against cells.

### CITATION LIST

#### Patent Literature

Patent Literature 1: Japanese Unexamined Patent Application Publication No. 2012-105587

### SUMMARY OF INVENTION

#### Technical Problem

In the technique described in Patent Literature 1, an antibody specifically to be adsorbed to a target cell is exposed on a hydrophobic surface. Then, when a solution containing target cells is brought into contact with this surface, the target cells in the solution are specifically adsorbed to the antibodies. After the target cells are adsorbed to the antibodies, a polymer film is immersed in water to make the surface hydrophilic. Thereby, the target cells are released from the antibodies so as to be separated and collected from the solution.

However, depending on the usage of the cell support described in Patent Literature 1, a solution to be brought into contact with the cell support contains cells other than target cells in some cases. Since cells exhibit hydrophobicity, the cells other than the target cells may be non-specifically adsorbed to other portions of the surface than those the antibodies located, that is, non-specifically adsorbed to hydrophobic portions of the surface, while the target cells are adsorbed to the antibodies. Thus, if removal of such non-specifically adsorbed cells is demanded, an agent, for example, a nonaqueous solvent or the like, has to be used in some cases. The use of the nonaqueous solvent may possibly affect the target cells adsorbed to the antibodies.

The present invention has been made to solve such problems as described above. An object of the present invention is to provide a cell separation and collection membrane capable of suppressing non-specific adsorption of celles while specifically adsorbing target cells, a culturing sheet and a culturing apparatus which use the membrane, and a cell separation and collection method.

#### Solution to Problem

To solve the above problems, the present inventors have earnestly studied. As a result, the inventors have found out that the problems can be solved by introducing a hydrophilic site to a side chain of a polymer compound that is fixed to a substrate and bound to an antibody.

## Advantageous Effects of Invention

According to the present invention, it is possible to provide a cell separation and collection membrane capable of suppressing non-specific adsorption of cells while specifically adsorbing target cells, a culturing sheet and a culturing apparatus which use the membrane, and a cell separation and collection method.

## BRIEF DESCRIPTION OF DRAWINGS

FIG. 1 is a perspective view of a separation and collection membrane of the present embodiment.
FIGS. 2A and 2B illustrate a mechanism of the separation and collection membrane of the present embodiment. FIG. 2A shows a state before heating, and FIG. 2B shows a state after heating.
FIG. 3A shows a modified example of the separation and collection membrane of the present embodiment, and FIG. 3B shows another modified example of the separation and collection membrane of the present embodiment.
FIG. 4 is a top view of a culturing sheet configured including the separation and collection membrane of the present embodiment.
FIG. 5 is a cross-sectional view of the culturing sheet of the present embodiment.
FIG. 6 is a block diagram of a culturing apparatus configured including the separation and collection membrane of the present embodiment.
FIG. 7A is an electron microphotograph taken when cells are adsorbed to the separation and collection membrane. FIG. 7B is an electron microphotograph of the separation and collection membrane taken after the adsorbed cells were collected. FIG. 7C is a drawing schematically illustrating the photograph of FIG. 7A. FIG. 7D is a drawing schematically illustrating the photograph of FIG. 7B.

## EMBODIMENTS FOR CARRYING OUT INVENTION

Hereinafter, the present invention will be described specifically on the basis of specific embodiments for carrying out the present invention (i.e., present embodiments).

[1. Cell separation and collection membrane]

[Configuration]

A cell separation and collection membrane of a present embodiment (hereinafter simply referred to as "separation and collection membrane" as appropriate) includes: a support; a hydrophobic polymer site fixed to the support and having a changeable structure in accordance with a temperature; a cell adsorbing site bound to the polymer site, exposed relative to an outer surface of the support, and specifically adsorbing a target cell when the cell adsorbing site is brought into contact with a treatment solution containing the target cells; and a hydrophilic site exposed relative to the outer surface and to be brought into contact with the treatment solution.
First, structures and functions of those components will be described. Then, how those components are bound to each other will be described with reference to the drawings.

(Support)

A support included in the separation and collection membrane is configured to fix a polymer site and so on described later. Such a support may be any support but is preferably one capable of chemically fixing a hydrophobic polymer site or the like. To be more specific, the support preferably includes at least one of a polymer material and a glass material. The use of such materials particularly allows a surface treatment for fixing the polymer site, etc. on the surface to be easily performed, and also provides an advantage of excellent durability when pressured in the surface treatment.
Examples of the polymer material include polystyrenes, polymethyl methacrylates, surface-modified agarose gels, and the like. Nevertheless, the support does not necessarily have to be configured of these materials, as long as the polymer site and so on can be fixed thereon. It is possible to use, for example, metal materials such as metal elements and metal oxides.
The support may have any surface shape, as long as the polymer site and so on can be fixed thereon. For example, the surface may be in a flat shape. Besides, a surface of the support can have a pillar structure, a spherical structure, a porous shape, or the like.

(Polymer Site)

The polymer site fixed to the support has a structure changeable in accordance with a temperature. To be more specific, for example, while a structure of the polymer site is maintained at room temperature, the structure collapses at or above a predetermined temperature higher than room temperature. Although the details will be described later, such a structural change makes it possible to embed a cell adsorbing site, which is bound to the polymer site, into an environment of a hydrophilic site described later. This facilitates the target cells to be released from the separation and collection membrane.

A specific structure of the polymer site is not particularly limited, as long as the structure changes in accordance with a temperature. Nevertheless, at least a part of the polymer site preferably has a spiral structure. When the polymer site includes a spiral structure, hydrogen bonds by which the spiral structure is kept can be cleaved depending on a temperature, allowing a structural change to occur easily. Further, the polymer site is preferably a hydrophobic polymer site. In other words, a polymer constituting the polymer site is preferably a hydrophobic polymer.

Moreover, the polymer site may be fixed to the support by any method. Examples of the method include those utilizing chemical bonds (such as covalent bond), physical adsorption (such as adsorption utilizing antigen-antibody reaction), and the like.

Specifically, a preferable example of the polymer site includes a polyamino acid. A polyamino acid is formed by polymerizing two or more amino acids. In this event, the amino acids are normally polymerized in a linear chain form. A polyamino acid has a spiral structure with hydrogen bonds formed in the molecule. Thus, the use of a polyamino acid provides an advantage in that the above-described structural change is likely to occur. Further, since a polyamino acid is normally in a linear chain form, it is easier to control the orientation of the polymer site. This facilitates the fixation of the hydrophobic site to a desired position on the support. Moreover, there is also an advantage that it is easy to bind a cell adsorbing site described later to the polymer site.

The polyamino acid may be constituted of only one type of amino acids (i.e., homopolymer), or may be constituted of two or more types of amino acids at any mixing ratio and in any combination (i.e., copolymer). The amino acid constituting the polyamino acid is not particularly limited. Examples thereof include glutamic acid, aspartic acid, asparagine, lysine, glutamine, cysteine, alanine, leucine, and the like.

Note that a number average molecular weight of the polyamino acid is not particularly limited, but is normally 5 kDa or more, preferably 10 kDa or more, and more preferably 50 kDa or more. When having the number average molecular weight within this range, the polyamino acid is more likely to be fixed to the support.

(Cell Adsorbing Site)

The cell adsorbing site is capable of specifically adsorbing a target cell when the cell adsorbing site is brought into contact with a treatment solution containing the target cells. Thus, the cell adsorbing site may be appropriately determined depending on a target cell targeted in the separation and collection. Particularly, an antibody targeting an antigen of the target cell is preferably used as the cell adsorbing site. The use of an antibody makes it possible to utilize a biological reaction of an antigen-antibody reaction to cells of a living tissue. The use of an antibody can make the target cells specifically adsorbed, and the desorption of the target cells easily controled.

Further, the cell adsorbing site is bound to the polymer site. The cell adsorbing site and the polymer site may be directly bound to each other, but are preferably bound to each other via a linker. Binding the cell adsorbing site to the hydrophobic polymer site via a linker can more surely lead to such a structure that the cell adsorbing site protrudes relative to the surface of the separation and collection membrane toward the outer side (i.e., the cell adsorbing site is exposed relative to the outer surface of the membrane). This can facilitate the cell adsorbing site to more specifically adsorb the target cell, when the separation and collection membrane is brought into contact with a treatment solution containing the target cells.

The linker is preferably a biotin-avidin linker. The use of a biotin-avidin system can further facilitate binding between the cell adsorbing site (for example, antibody) and the hydrophobic polymer site (for example, polyamino acid). More specifically, biotinylation of both the cell adsorbing site and the polymer site together with the use of avidin can facilitate binding between the cell adsorbing site and the polymer site via avidin of the biotin-avidin system.

Further, as a linker, a domain constituting protein A or protein G may be used. Specifically, the polymer site and the cell adsorbing site may be bound to protein A or protein G serving as an antigen. This enables the binding between the polymer site and the cell adsorbing site via an IgG (immunoglobulin) antibody, and produces a similar effect to that obtained when the biotin-avidin system is used.

A specific position to which the cell adsorbing site is bound in the polymer site is not particularly limited. Nevertheless, although the details will be described later, the cell adsorbing site is preferably bound to a terminal of a polymer constituting the polymer site, from the viewpoint that the cell adsorbing site is more surely exposed relative to the outer surface of the separation and collection membrane. Specifically, for example, in a case where the polymer

constituting the polymer site is in a linear chain form, the cell adsorbing site may be located at a terminal of the straight chain. Or, in a case where the polymer is in a reticulated form, the cell adsorbing site is located at a terminal of the reticulated structure. Binding the cell adsorbing site at the above positions can make the cell adsorbing site more surely exposed relative to the outer surface of the separation and collection membrane.

As described above, the cell adsorbing site is exposed relative to the outer surface of the separation and collection membrane. This makes it possible to specifically bind a target cell to the cell adsorbing site and then collect the target cells when a treatment solution is brought into contact with the separation and collection membrane. The method for exposing the cell adsorbing site relative to the outer surface is not particularly limited. For example, the cell adsorbing site can be exposed relative to the outer surface by binding the cell adsorbing site to the polymer site via the linker as described above.

Further, by setting positions (i.e., heights) of portions other than the cell adsorbing sites lower than a position (i.e., height) of the cell adsorbing site, the cell adsorbing site can be exposed relative to the outer surface without including a linker. Note that those methods for exposing the cell adsorbing site relative to the outer surface are merely examples. Any method may be employed, and the cell adsorbing site may be in any form, as long as the cell adsorbing site is exposed relative to the outer surface of the separation and collection membrane.

As having been described above, exposing the cell adsorbing site relative to the outer surface of the membrane and binding the cell adsorbing site to the polymer site make it possible to reduce a non-specifically adsorbed area on the surface of the separation and collection membrane. In other words, the cell adsorbing sites are arranged with covering the polymer sites in the present invention. This arrangement makes it possible to reduce the non-specifically adsorbed area of the membrane resulting from the hydrophobic polymer site.

In general, cells are likely to be non-specifically adsorbed to a hydrophobic area. Hence, if there is a hydrophobic area on the surface of the separation and collection membrane, cells other than target cells are likely to be non-specifically adsorbed to the hydrophobic area. On the other hand, the cell adsorbing site is exposed relative to the outer surface and bound to the polymer site in the present embodiment. This configuration makes it possible to specifically adsorb a target cell to the cell adsorbing site. Further, since the hydrophobic area is reduced as described above, it is also possible to suppress non-specific adsorption of cells other than the target cells to the separation and collection membrane.

(Hydrophilic Site)

The hydrophilic site is a site exposed relative to the outer surface of the separation and collection membrane, and to be brought into contact with (for example, impregnated with) a treatment solution containing target cells when brought into contact with the treatment solution. Arrangement of the hydrophilic site to be exposed relative to the outer surface and located at an area where the polymer site and the cell adsorbing site are not present makes it possible to further suppress the non-specific cell adsorption.

Examples of the hydrophilic site include ones having hydrophilic functional groups, specifically, a hydroxyl group, a carboxyl group, or the like. More specific examples of the hydrophilic site include ethylene glycol and polymers thereof (i.e., polyethylene glycols), molecules having a phospholipid structure, phosphoric acid and polymers thereof (i.e., polyphosphoric acids), and the like. Among those, the hydrophilic site preferably includes a polyethylene glycol in which two or more ethylene glycols are polymerized, from the viewpoints of favorable hydrophilicity, binding affinity to the polymer site, and so forth.

Further, the hydrophilic site may be provided independently separated from the polymer site. Nevertheless, from the viewpoint of further promoting the structural change of the hydrophobic site, the hydrophilic site is preferably bound to the polymer site. In other words, the hydrophilic site is preferably integrated with the polymer site. Binding the hydrophilic site to the hydrophobic polymer site can further promote the structural change of the polymer site in accordance with a temperature change. However, it is not always required that all of such hydrophilic sites to be provided to the separation and collection membrane should be bound to the polymer sites. Binding a hydrophilic group or the like as appropriate makes it possible to control the temperature response of the polymer site.

A position where the hydrophilic site binds in the polymer site is not particularly limited. Nevertheless, in the case where the polymer site is in a linear chain form, the hydrophilic site is preferably bound to a side chain of the polymer site. This allows the hydrophilic sites to be easily arranged to an environment of the polymer sites in the separation and collection membrane. Hence, the hydrophilic site can be more surely exposed relative to the outer surface of the separation and collection membrane.

As described above, the polymer site is preferably a polyamino acid, and the hydrophilic site preferably contains polyethylene glycol. Additionally, the hydrophilic site is preferably bound to the polymer site. A material preferable as the polymer and hydrophilic site satisfying those conditions is, for example, a compound represented by the following Formula (1).

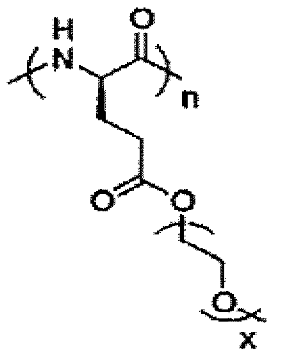

・・・Formula (1)

* In Formula (1), n and x are each independently an integer of 2 or more.

[Mechanism]

Next, the configuration and mechanism of the separation and collection membrane of the present embodiment will be described in more details with reference to the drawings.

FIG. 1 is a perspective view of a separation and collection membrane 10 of the present embodiment. It is shown in a macroscopic scale of the separation and collection membrane 10 that a thin film 2 is formed on a surface of a support 1. This thin film 2 includes the above-described polymer sites, cell adsorbing sites, and hydrophilic sites.

FIGS. 2A and 2B illustrate a mechanism of the separation and collection membrane 10 of the present embodiment. FIG. 2A shows a state before heating, and FIG. 2B shows a state after heating. In the present embodiment, heating the separation and collection membrane changes (i.e., destroys) a structure of the polymer site. As shown in FIG. 2A, the thin film 2 of the present embodiment includes spiral polymer sites 2a, cell adsorbing sites 2b bound to the polymer sites 2a, and hydrophilic sites 2c bound to the polymer sites 2a. The cell adsorbing sites 2b are respectively bound to the polymer sites 2a (i.e., three in the illustrated example). Further, the polymer sites 2a and the hydrophilic sites 2c are alternately arranged relative to a surface of the support 1. In other words, the surface of the support 1 except for the area where the polymer site 2a is covered with the cell adsorbing site 2b is covered with the hydrophilic sites 2c.

Further, in the present embodiment, after target cells 20 are adsorbed to the cell adsorbing sites 2b (FIG. 2A), heating the separation and collection membrane 10 changes the structure of the polymer sites 2a and releases the target cells adsorbed to the cell adsorbing sites 2b (FIG. 2B). Note that, in the illustrated example, one of the target cells 20 is adsorbed to one of the cell adsorbing sites 2b for convenience of the illustration. Nevertheless, the single target cell 20 may be adsorbed to two or more cell adsorbing sites 2b.

The mechanism during the change from the state in FIG. 2A to the state in FIG. 2B will be described. Before heating, the spiral structure of the polymer site 2a is maintained. In addition, the target cell 20 is adsorbed to the cell adsorbing site 2b. Then, when the separation and collection membrane 10 is heated in this state, the spiral structure of the polymer site 2a is broken by the thermal energy and changed to a random structure, and the cell adsorbing site 2b exposed relative to the outer surface of the support is embedded in the environment of the hydrophilic sites 2c. Thereby, the adsorption of the target cell 20 to the cell adsorbing sites 2 is blocked by steric hindrance of the hydrophobic polymer sites 2a having changed to the random structure. Further, when the cell adsorbing site 2b are embedded in the environment of the hydrophilic sites 2c, this results in a state where the target cells 20 are put on the hydrophilic sites 2c covering the support 1. Hence, the hydrophobic target cells 20 can be easily collected from the environmrnt of the hydrophilic sites 2c.

The temperature at which the state in FIG. 2A is changed to the state in FIG. 2B can be settable, for example, associated with the structure of the polymer site 2a. For example, in a case where the hydrophobic polymer site 2a has a spiral structure in a long length, the number of hydrogen bonds is also large, so that thermal energy required for cleaving the hydrogen bonds needed for changing the structure comes to be large. Hence, the temperature for causing the structural change tends to be high. On the other hand, in a case where the spiral structure included is in a short length, the number of hydrogen bonds comes to be small. Hence, the temperature for causing the structural change tends to be low. Further, when the hydrophilic sites 2c are bound to the polymer site 2a, the temperature may be also settable associated with the structure of the hydrophilic sites 2c.

Note that, in the above description, the spiral structure is changed to the random structure at a predetermined temperature or higher. Nonetheless, it is acceptable that the random structure is maintained than a predetermined temperatur, but is changed to the spiral structure at a predetermined temperature or higher. In this case, cooling causes the structural change, so that the target cells 20 are brought into a collectable state.

Note that the separation and collection membrane of the present embodiment is not limited to the embodiments

of the illustration and the above descriptions. For example, the cell adsorbing sites 2b do not have to be bound to all the hydrophobic polymer sites 2a, and a separation and collection membrane 10A as shown in FIG. 3A may be formed in which the cell adsorbing sites 2b are bound only to a part of the hydrophobic polymer sites 2a. In this way, even when the number of target cells contained in a treatment solution is small, it is possible to efficiently collect the target cells.

Alternatively, a separation and collection membrane 10B as shown in FIG. 3B may be formed including, in addition to the hydrophilic sites 2c bound to the polymer sites 2a, other hydrophilic sites 2d provided at an area where the support 1 is exposed between the adjacent environments of the hydrophilic sites 2c. In this way, it is possible to more surely prevent the non-specific adsorption of cells.

[2. Method for Producing Cell Separation and Collection Membrane]

Next, a method for producing the separation and collection membrane of the present embodiment will be described. The method for producing the separation and collection membrane of the present embodiment is not particularly limited. The separation and collection membrane can be produced, for example, by the following method. Note that, in the following descriptions, an example of the method for producing the separation and collection membrane will be described with reference to FIG. 2A, in which hydrophilic sites are bound to polymer sites.

First, the polymer sites 2a to which the hydrophilic sites 2c are bound (i.e., which can be prepared by any method) are fixed to the surface of the support 1, for example, by spin-coating, a dipping method, a vapor deposition method, or the like. Herein, as the fixing method, preferable one is a reaction of chemical bonding which does not allow the polymer sites 2a themselves to be released by the reversible reaction. More specifically, examples of the chemical bond include an amide bond, an ester bond, a silanol bond, a maleimide, a thioester bond, a chemical bond formed by the Diels-Alder reaction, and a chemical bond formed by click reactions, or depending on the usage, a gold-thiol bond, an imine bond, and the like.

Here, when fixing the polymer sites 2a, the hydrophilic sites 2c may be fixed or may not be fixed onto the support 1. Nevertheless, once the polymer sites 2a are fixed onto the support 1, the hydrophilic sites 2c bound to side chains of the polymer sites 2a can be sufficiently disposed relative to the surface of the support 1 without being fixed onto the support 1. The unfixed polymer sites 2a are then removed with a good solvent.

Subsequently, the cell adsorbing sites 2b are bound by chemical binding to ends of the polymer sites 2a thus fixed. In this event, the cell adsorbing sites 2b do not always have to be bound to the ends of the hydrophobic polymer sites 2a. Thus, the cell adsorbing sites 2b may be bound to any portion of the polymer sites 2a, as long as the cell adsorbing sites 2b are exposed relative to the outer surface of the cell separation and collection membrane 10.

In this way, the separation and collection membrane can be produced in which the hydrophilic sites 2c are exposed relative to the surface of the membrane.

Note that, when binding the polymer sites 2a to the support 1, the polymer sites 2a may be dispersed where necessary in a solvent to coat therewith the surface of the support 1 by various methods such as, for example, spin-coating, spray-coating, dip-coating, roll-coating, and bead-coating. The hydrophobic polymer sites 2a can also be disposed to the support 1 by such methods.

[3. Use of Cell Separation and Collection Membrane]

Next, use of the separation and collection membrane of the present embodiment will be described with reference to the drawings. The separation and collection membrane of the present embodiment is applicable to any use, but preferably to, for example, biodevices such as a cell culturing sheet, a cell culturing apparatus, and the like. Hereinafter, two examples among those will be described as preferable use of the separation and collection membrane of the present embodiment.

[3-1. Cell Culturing Sheet]

FIG. 4 is a top view of a culturing sheet 50 configured including the separation and collection membrane of the present embodiment. The cell culturing sheet 50 of the present embodiment (hereinafter simply referred to as "culturing sheet 50" as appropriate) has the same configuration as that of the separation and collection membrane 10 described above. The cell culturing sheet 50 includes: the support 1 mainly made of polystyrene and having a thickness of 0.5 $\mu$m; and multiple cylindrical fine protrusions 2A (hereinafter simply referred to as "protrusions 2A") provided upright on this thin film, and mainly made of polystyrene. The protrusions 2A have a cylindrical shape with a diameter of 500 nm and a height of 1 $\mu$m. The protrusions 2A are arranged at intervals (i.e., pitches) of 1 $\mu$m. Additionally, in the culturing sheet 50 of the present embodiment, the protrusions 2A are first arranged in a cylindrical shape, and then the protrusions 2A disposed in a cross shaped area are removed, so that a cross shaped clearance area 51 is formed on the support 1.

The protrusions 2A can be formed by the following method. Specifically, first, protrusions made of polystyrene

and having the aforementioned shape serving as a base part are formed on the support 1 by an imprinting method. Then, a surface of the base part is subjected to a surface oxidation treatment using an ultraviolet ray/ozone generator. Finally, as shown in FIG. 2A, the polymer member and so forth are bound to the base part having been subjected to the surface oxidation treatment. Thus, the culturing sheet 50 including the protrusions 2A capable of adsorbing target cells is obtained.

The culturing sheet 50 is impregnated with a culture solution in a container such as a glass Petri dish in which the culture solution having been introduced. The target cells 20 (hereinafter simply referred to as "cells 20") are cultured on the culturing sheet 50. A specific culturing method and a method for using the culturing sheet 50 (i.e., cell culturing method) will be described with reference to FIG. 5.

FIG. 5 is a cross-sectional view of the culturing sheet 50 of the present embodiment. The cells 20 are adsorbed (i.e., fixed) to the cell adsorbing sites 2b (not shown in FIG. 5) on the surfaces of the protrusions 2A. Note that, the illustrated example shows a state where one cell 20 is adsorbed to the cell adsorbing sites 2b on the multiple protrusions 2A. Then, a culture solution such as a medium and nutrients is supplied while the cell 20 is adsorbed as described above, to thereby culture the cell 20. Note that the cells 20 which can be cultured are not particularly limited, and examples thereof include cells (or tissues) of skin, bone, and blood or the like.

As shown in FIG. 5, the support 1 and the cell 20 are in a state where they are apart from each other. Hence, the culture solution and oxygen for culturing the cell 20 are supplied from both upper and lower sides of the cell 20. This makes it possible to efficiently culture the cell 20. Further, waste products (for example, carbon dioxide), which are discharged when culturing the cell 20, are efficiently discharged from the upper and lower sides of the cell 20.

When such a culturing sheet 50 is used, the cell 20 is supported only by top surfaces of the protrusions 2A. This makes it possible to reduce a contact area of the cell 20 compared to a contact area of the cell 20 which has been conventionally cultured on a bottom surface of a glass Petri dish. Thus, a damage of the cell 20 can be greatly reduced when the cell 20 is released. This can enhance the colonization percentage on a transplantation site when the cell 20 is transplanted.

Further, as shown in FIG. 5, the culture solution readily flows to the entire cell 20 through the space formed by the protrusions 2A under the cell 20. As a result, it is possible to efficiently supply the cell 20 with nutrients and discharge waste products of the cell 20, so that the death of the cell 20 during the culturing, which has otherwise occurred conventionally, can be suppressed.

Note that the protrusions 2A may be obtained by subjecting a polymer material to a hydrophilication treatment by a plasma treatment or the like. Further, as the polymer material, it is preferable to select a material affecting little on cells (or tissues) to be cultured. More specificaly, polystyrene is used, but the polymer material is not limited thereto. For example, polymethyl methacrylate, polylactic acid, and the like are also preferable as the polymer material.

Moreover, an example of the biodevices besides the above-described one includes particularly μTAS generally called medical and diagnostic tools. Specific examples thereof include biodevices having a finely processed surface, and ones used for detection, synthesis, and other means in the medical and chemical fields.

[3-2. Cell Culturing Apparatus]

A cell culturing apparatus 100 of the present embodiment (hereinafter simply referred to as "culturing apparatus 100" as appropriate) includes the separation and collection membrane 10. Further, this culturing apparatus 100 is configured to separate and collect target cells contained in a treatment solution. Hereinafter, descriptions will be given of a specific configuration of the culturing apparatus 100 and a cell separation and collection method using the culturing apparatus 100.

FIG. 6 is a block diagram of the culturing apparatus 100 configured including the separation and collection membrane of the present embodiment. The culturing apparatus 100 includes: the separation and collection membrane 10; an impregnating device 102b (or contacting device) configured to impregnate the separation and collection membrane 10 (i.e., to bring the separation and collection membrane 10 into contact) with a treatment solution containing target cells; a heating device 104 (or heating-cooling device) configured to heat the separation and collection membrane 10; and a cell collecting device 105 configured to collect the target cells adsorbed to the separation and collection membrane 10.

In addition, besides the above devices, the culturing apparatus 100 includes: a washing liquid 100b for washing out cells non-specifically adsorbed to the separation and collection membrane; a medium reagent 100c for culturing the target cells; a cool box 100a configured to store the medium reagent 100a; and a liquid delivery system 101 configured to supply a thermostatic chamber 102 with the washing liquid 100b and the medium reagent 100c. Further, the thermostatic chamber 102 included in the culturing apparatus 100 is configured to impregnate the separation and collection membrane 10 with a culture solution containing target cells in a culturing device 102a housed inside the thermostatic chamber 102. Moreover, the atmosphere in the culturing device 102a is controlled by a gas exchanging device 103.

Next, the cell separation and collection method using the culturing apparatus 100 will be described. Note that,

in the following descriptions, cells are adsorbed to the separation and collection membrane, cultured, and then collected. Nevertheless, cells may be collected immediately after the adsorption without culturing.

First, as shown in FIG. 6, the culturing device 102a is supplied with a treatment solution containing target cells. Then, the impregnating device 102b impregnates the separation and collection membrane 10 (i.e., brings the separation and collection membrane 10 into contact) with the treatment solution containing the target cells, thereby adsorbing the target cells in the treatment solution to the separation and collection membrane (i.e., contacting step of the separation and collection membrane). Subsequently, the separation and collection membrane 10 is washed with the washing liquid 100b to wash out non-specifically adsorbed cells, whereby specifically adsorbed target cells are to be cultured. In this way, the target cells can be efficiently cultured.

Thereafter, in the impregnating device 102b, the impregnated separation and collection membrane 10 is taken out from the treatment solution. After that, the heating device 104 heats the separation and collection membrane 10 (i.e., heating-cooling step). Thereby, the structure of the polymer sites 2a of the separation and collection membrane 10 is changed, and the specifically adsorbed target cells (i.e., cultured target cells) can be released easily. Then, the surface of the separation and collection membrane 10 in this state is supplied with a cell collecting liquid 105a by the cell collecting device 105. Thereby, the cultured target cells are released from the surface of the separation and collection membrane 10. The released target cells are collected by the cell collector 105.

The above-describe method makes it possible to suppress a damage of cells when being released, which has otherwise occurred conventionally when a normal medium or a conventional culturing sheet is used, and to reduce the use of a chemical during the collection. Thus, it is possible to suppress a decrease in the collection rate of target cells.

Note that, in the above-described example, the target cells are released by heating. Nonetheless, through the change in the structure of the hydrophobic polymer site 2b, the target cells may be released by cooling. In this case, a cooling device configured to cool the separation and collection membrane 10 should be employed in place of the heating device 104.

EXAMPLES

Hereinafter, the present invention will be described in more details based on Examples.

[Preparation of Cell Separation and Collection Membrane, and Evaluation of Cell Selectivity]

<Examples 1 to 3>

(Preparing PEG-Modified Polylysine(s))

First, 1-ethyl-3-carbodiimide (WSC), *N*-hydroxysuccinimide (NHS), and a polyethylene glycol having a carboxyl terminal group (i.e., in Formula (1), x=3) were dissolved in pure water. This solution was cooled to 4°C, and polylysines (i.e., a mixture with various molecular weights) were added thereto with stirring. Then, the resultant mixture was stirred at normal temperature for 2 hours. Thus, PEG-modified polylysines were obtained in each of which the polyethylene glycol (i.e., hydrophilic site) was bound to a side chain of the polylysine (i.e., polymer site).

Next, gel filtration chromatography was employed to remove unreacted low-molecular-weight substances, and to separate three types of PEG-modified polylysine: one having a number average molecular weight of 5 kDa or more (Example 1), one having a number average molecular weight of 12 kDa or more (Example 2), and one having a number average molecular weight of 25 kDa or more (Example 3). Hereinbelow, unless otherwise particularly stated, the term "polylysine(s)" means the "PEG-modified polylysine(s)" thus prepared.

(Fixing Polylysine(s) to Support)

Each of the polylysines of Examples 1 to 3 was covalently bonded on a resin plate (e.g., support made of polystylene) according to the following procedure. Herein, the resin plate had been subjected to an amino group treatment on a surface thereof. To a 1 mol% aqueous solution of each polylysine, 1-ethyl-3-carbodiimide (WSC) and N-hydroxysuccinimide (NHS) were added, and each plate was immersed in the resulting solution. Then, the reaction was allowed to take place at room temperature for 2 hours. Thus, a support to which the polylysine was bound was obtained. Subsequently, the respective resin plates were washed three times with pure water.

(Binding Protein A to Polylysine(s))

The three plates to which the polylysines of Examples 1 to 3 were respectively bound were immersed in 2% glutaraldehyde having been diluted with a carbonate buffer solution, and left standing at 37°C for 2 hours to carry out

the reaction. After the reaction, each of the plates was washed three times with phosphate-buffered saline (PBS). Then, an aqueous solution of protein A was prepared at a concentration of 0.1 wt% by using a PBS buffer solution having a pH value of 7.4. Each plate was immersed in this protein A solution, thereby binding the protein A to the polylysine.

(Binding CD40 Antibody)

Subsequently, a CD40 antibody was bound to the protein A (i.e., antigen) fixed to each plate according to the following procedure. The CD40 antibody is an antibody specifically bound to Chinese hamster ovary cells. First, each plate was washed three times with a wash buffer (200 μl). Then, 50 μl of immunoglobulin G (IgG) was added and incubated at 4°C overnight. Thereby, the IgG was bound to the protein A. Subsequently, while the temperature was kept at 4°C, each plate was washed three times using a wash buffer (200 μl). Thereafter, each plate was immersed in a solution of the CD40 antibody, so that the CD40 antibody was bound to the IgG. By the above operations, separation and collection membranes of Examples 1 to 3 were prepared.

(Evaluating Cell Adsorption Selectivity)

An aqueous buffer solution containing Chinese hamster ovary cells (CHO cells) and mesenchymal stem cells (MSCs) at the rate of 1:1 was added dropwise to the separation and collection membranes of Examples 1 to 3 at 4°C, followed by incubation. The MSCs were not adsorbed to the CD40 antibody of the separation and collection membranes, whereas the CHO cells were specifically adsorbed thereto. Then, each plate was washed three times with an aqueous buffer solution to wash out the MSCs which were non-specifically adsorbed. The MSCs thus washed out were collected, and the cell count was verified with a flow cytometer. Note that the CHO cells and the MSC cells were distinguished from each other with the flow cytometer by fluorescence-labeling the MSC cells with a FITC-labeled anti-CD105 antibody.
Next, each plate was heated to 37°C in an aqueous buffer solution to change the structure of the polylysine, thereby releasing the specifically adsorbed CHO cells. The released CHO cells were collected using an aqueous buffer solution. Then, the cell count of the collected CHO cells was verified with the flow cytometer.
Based on the cell count of the MSCs and the cell count of the CHO cells thus collected, a cell selectivity ratio was calculated using the following equation (1).

(Cell selectivity ratio) = (the number of cells collected with the separation and collection membrane) / (the number of cells before collection) × 100[%] ··· Equation (1)

<Example 4>

A cell selectivity ratio was evaluated in the same manner as in Example 1, except that an aqueous buffer solution containing B cells and MSCs at the rate of 1:1 was used. Note that the MSCs were not adsorbed to the CD40 antibody on the separation and collection membrane, whereas the B cells were specifically adsorbed thereto.

<Example 5>

A selectivity ratio was evaluated in the same manner as in Example 1, except that an aqueous buffer solution containing circulating tumor cells (CTCs) and MSCs at the rate of 1:1 was used. Note that the MSCs were not adsorbed to the CD40 antibody on the separation and collection membrane, whereas the CTCs were specifically adsorbed thereto.
<Comparative Example 1>
A separation and collection membrane of Comparative Example 1 was prepared the same as in Example 1, except that the "polyethylene glycol having a carboxyl terminal group " was not used (i.e., no hydrophilic site was incorporated), and that a polylysine (i.e., polylysine incorporating no hydrophilic site) had a number average molecular weight of 7 kDa. Then, the cell selectivity ratio of this separation and collection membrane was evaluated the same as in Example 1.

<Evaluation Results>

Table 1 shows the cell selectivity ratios of Examples 1 to 5 and Comparative Example 1.

[Table 1]

| | Presence or absence of hydrophilic site | Number average molecular weight | Target cells | Cell selectivity ratio |
|---|---|---|---|---|
| Example 1 | present | 5 kDa or more | CHO cells | 91% |
| Example 2 | present | 12 kDa or more | CHO cells | 94% |
| Example 3 | present | 25 kDA or more | CHO cells | 93% |
| Example 4 | present | 5 kDA or more | B cells | 90% |
| Example 5 | present | 5 kDA or more | CTCs | 91% |
| Comparative Example 1 | absent | 7 kDA or more | CHO cells | 48% |

As shown in Table 1, all of the separation and collection membranes incorporating the hydrophilic sites (Examples 1 to 5) exhibited favorable cell selectivity ratios regardless of the number average molecular weight (i.e., size) and the type of target cells. Thus, it is revealed that incorporating a hydrophilic site in a cell separation and collection membrane suppresses non-specific cell adsorption, realizing the efficient separation and collection of target cells.

On the other hand, in Comparative Example 1 incorporating no hydrophilic site, although the number average molecular weight and the type of target cells were almost the same as those of Example 1, the cell selectivity ratio was approximately the half of Example 1. Thus, it is revealed that the use of the separation and collection membrane incorporating no hydrophilic site increases non-specific adsorption, resulting in the significant decrease of the cell selectivity ratio.

Further, FIGS. 7A and 7B respectively show electron microphotographs of a surface of the separation and collection membrane of Example 1 taken after the cells were adsorbed thereto, and a surface thereof taken after the cells were separated and collected (i.e., released).

FIG. 7A is an electron microphotograph taken when the cells were adsorbed to the separation and collection membrane. FIG. 7B is an electron microphotograph e taken when the adsorbed cells were collected from the separation and collection membran. FIG. 7C is a drawing schematically illustrating the photograph of FIG. 7A, and FIG. 7D is a drawing schematically illustrating the photograph of FIG. 7B. It is revealed that although the cells were adsorbed to the surface of the separation and collection membrane as shown in FIG. 7A, heating the separation and collection membrane completely released the cells from the surface of the separation and collection membrane as shown in FIG. 7B.

[Cell Culturing Using Cell Separation and Collection Membrane]

The culturing sheet shown in FIGS. 4 and 5 was prepared by the same method as in Example 1. The prepared culturing sheet was put into a glass Petri dish in which a culture solution had been poured. Normal human epidermal keratinocytes were cultured on the culturing sheet. In this culturing, HuMedia-KB2 manufactured by Kurabo Industries Ltd. was used as a medium, and the culturing temperature was set at 37°C. The culturing was performed under a 5% carbon dioxide flow.

As a result, the normal human epidermal keratinocytes were normally attached onto the culturing sheet and grew in the sheet shape. On day 14 after the start of culturing, the cells in the sheet shape were released, thereby to obtain the sheet-shaped epidermal keratinocytes with few damages.

**Reference Signs List**

| | |
|---|---|
| 1 | support |
| 2 | thin film |
| 2a | polymer site |
| 2b | cell adsorbing site |
| 2c | hydrophilic site |

50 culturing sheet
100 culturing apparatus
102b impregnating device
104 heating device
105 cell collecting device
105a cell collecting liquid

**Claims**

**1.** A cell separation and collection membrane comprising:

a support;
a polymer site fixed to the support and having a changeable structure in accordance with a temperature;
a cell adsorbing site bound to the polymer site, exposed relative to an outer surface of the support, and specifically adsorbing a target cell when the cell adsorbing site is brought into contact with a treatment solution containing the target cells; and
a hydrophilic site exposed relative to the outer surface and to be brought into contact with the treatment solution.

**2.** The cell separation and collection membrane according to claim 1, wherein at least a part of the polymer site includes a spiral structure.

**3.** The cell separation and collection membrane according to claim 1 or 2, wherein the hydrophilic site is bound to the polymer site.

**4.** The cell separation and collection membrane according to claim 1 or 2, wherein the polymer site comprises a polyamino acid.

**5.** The cell separation and collection membrane according to claim 1 or 2, wherein the hydrophilic site comprises polyethylene glycol.

**6.** The cell separation and collection membrane according to claim 1 or 2, wherein the cell adsorbing site is an antibody against an antigen of the target cell.

**7.** The cell separation and collection membrane according to claim 1 or 2, wherein the support comprises at least one of a polymer material and a glass material.

**8.** The cell separation and collection membrane according to claim 1 or 2, wherein a position of any one of the cell adsorbing sites is higher than a position of any one of the hydrophilic sites with respect to the support.

**9.** A cell culturing sheet provided with a cell separation and collection membrane, the cell separation and collection membrane comprising:

a support;
a polymer site fixed to the support and having a changeable structure in accordance with a temperature;
a cell adsorbing site bound to the polymer site, exposed relative to an outer surface of the support, and specifically adsorbing a target cell when the cell adsorbing site is brought into contact with a treatment solution containing the target cells; and
a hydrophilic site exposed relative to the outer surface and to be brought into contact with the treatment solution.

**10.** A cell culturing apparatus provided with a cell separation and collection membrane, the cell separation and collection membane comprising:

a support;
a polymer site fixed to the support and having a changeable structure in accordance with a temperature;
a cell adsorbing site bound to the polymer site, exposed relative to an outer surface of the support, and specifically adsorbing a target cell when the cell adsorbing site is brought into contact with a treatment solution containing the target cells; and

a hydrophilic site exposed relative to the outer surface and to be brought into contact with the treatment solution.

**11.** A cell separation and collection method using a cell culturing apparatus,
the apparatus comprising:

a cell separation and collection membrane comprising

a support,
a hydrophobic polymer site fixed to the support and having a changeable structure in accordance with a temperature,
a cell adsorbing site bound to the polymer site, exposed relative to an outer surface of the support, and specifically adsorbing a target cell when the cell adsorbing site is brought into contact with a treatment solution containing the target cells, and
a hydrophilic site exposed relative to the outer surface and to be brought into contact with the treatment solution;

a contacting device configured to bring the cell separation and collection membrane into contact with the treatment solution;
a heating-cooling device configured to heat or cool the cell separation and collection membrane; and
a cell collecting device configured to collect the target cells adsorbed to the cell separation and collection membrane,

the method comprising:

a cell separation and collection membrane contacting step of bringing the cell separation and collection membrane into contact with the treatment solution containing the target cells by using the contacting device, thereby having the target cells in the treatment solution adsorbed to the cell adsorbing sites;
a heating-cooling step of heating or cooling the cell separation and collection membrane thus brought into contact during the separation and collection membrane contacting step, at an outside of the treatment solution by using the heating-cooling device; and
a target cell collecting step of collecting the target cells adsorbed to the cell adsorbing sites, performed by supplying a membrane treatment solution to the surface of the cell separation and collection membrane thus heated or cooled in the heating-cooling step, and collecting the supplied membrane treatment solution containing the target cells with the cell collecting device.

**Amended claims under Art. 19.1 PCT**

**1.** (**Amended**) A cell separation and collection membrane comprising:

a support;
a polymer site fixed to the support and having a changeable structure in accordance with a temperature;
a cell adsorbing site bound to the polymer site, exposed relative to an outer surface of the support, and specifically adsorbing a target cell when the cell adsorbing site is brought into contact with a treatment solution containing the target cells; and
a hydrophilic site exposed relative to the outer surface and to be brought into contact with the treatment solution, wherein
a position of any one of the cell adsorbing sites is higher than a position of any one of the hydrophilic sites with respect to the support.

**2.** The cell separation and collection membrane according to claim 1, wherein at least a part of the polymer site includes a spiral structure.

**3.** The cell separation and collection membrane according to claim 1 or 2, wherein the hydrophilic site is bound to the polymer site.

**4.** The cell separation and collection membrane according to claim 1 or 2, wherein the polymer site comprises a polyamino acid.

5. The cell separation and collection membrane according to claim 1 or 2, wherein the hydrophilic site comprises polyethylene glycol.

6. The cell separation and collection membrane according to claim 1 or 2, wherein the cell adsorbing site is an antibody against an antigen of the target cell.

7. The cell separation and collection membrane according to claim 1 or 2, wherein the support comprises at least one of a polymer material and a glass material.

8. (**Canceled**)

9. (**Amended**) A cell culturing sheet provided with a cell separation and collection membrane, the cell separation and collection membrane comprising:

   a support;
   a polymer site fixed to the support and having a changeable structure in accordance with a temperature;
   a cell adsorbing site bound to the polymer site, exposed relative to an outer surface of the support, and specifically adsorbing a target cell when the cell adsorbing site is brought into contact with a treatment solution containing the target cells; and
   a hydrophilic site exposed relative to the outer surface and to be brought into contact with the treatment solution, wherein

   a position of any one of the cell adsorbing sites is higher than a position of any one of the hydrophilic sites with respect to the support.

10. (**Amended**) A cell culturing apparatus provided with a cell separation and collection membrane, the cell separation and collection membrane comprising:

   a support;
   a polymer site fixed to the support and having a changeable structure in accordance with a temperature;
   a cell adsorbing site bound to the polymer site, exposed relative to an outer surface of the support, and specifically adsorbing a target cell when the cell adsorbing site is brought into contact with a treatment solution containing the target cells; and
   a hydrophilic site exposed relative to the outer surface and to be brought into contact with the treatment solution, wherein
   a position of any one of the cell adsorbing sites is higher than a position of any one of the hydrophilic sites with respect to the support.

11. (**Amended**) A cell separation and collection method using a cell culturing apparatus,
   the apparatus comprising:

   a cell separation and collection membrane comprising:

      a support,
      a hydrophobic polymer site fixed to the support and having a changeable structure in accordance with a temperature,
      a cell adsorbing site bound to the polymer site, exposed relative to an outer surface of the support, and specifically adsorbing a target cell when the cell adsorbing site is brought into contact with a treatment solution containing the target cells, and
      a hydrophilic site exposed relative to the outer surface and to be brought into contact with the treatment solution, wherein
      a position of any one of the cell adsorbing sites is higher than a position of any one of the hydrophilic sites with respect to the support;

   a contacting device configured to bring the separation and collection membrane into contact with the treatment solution;
   a heating-cooling device configured to heat or cool the cell separation and collection membrane; and
   a cell collecting device configured to collect the target cells adsorbed to the cell separation and collection

membrane, the method comprising:

a cell separation and collection membrane contacting step of bringing the cell separation and collection membrane into contact with the treatment solution containing the target cells by using the contacting device, thereby having the target cells in the treatment solution adsorbed to the cell adsorbing sites;
a heating-cooling step of heating or cooling the cell separation and collection membrane thus brought into contact during the separation and collection membrane contacting step, at an outside of the treatment solution by using the heating-cooling device; and
a target cell collecting step of collecting the target cells adsorbed to the cell adsorbing sites, performed by supplying a membrane treatment solution to the surface of the cell separation and collection membrane thus heated or cooled in the heating-cooling step, and collecting the supplied membrane treatment solution containing the target cells with the cell collecting device.

**Statement under Art. 19.1 PCT**

Contents of amendment

(1) Claim 1 was limited by the subject matter of original Claim 8.
(2) Original Claim 8 was canceled.
(3) Claim 9 was limited by the structural feature disclosed in the description of the original specification and so on.
(4) Claim 10 was limited by the structural feature disclosed in the description of the original specification and so on.
(5) Claim 11 was limited by the structural feature disclosed in the description of the original specification and so on.

Explanation

(1)
The matter described from lines 7 to 8 in Claim 1 corresponds to that in original Claim 8.
(2)
The amendment of adding lines 8 to 9 in claim 9 is supported by paragraphs 0029, 0052, and 0055 of the originally filed specification and FIGS. 2A and 5.
(3)
The amendment of adding lines 8 to 9 in claim 10 is supported by paragraphs 0029, 0061, and 0062 of the originally filed specification and FIGS. 2A and 6.
(4)
The amendment of adding lines 5 to 7 in claim 11 is supported by paragraphs 0029, 0061, 0062, and 0064 of the originally filed specification and FIGS. 2A and 6.

As described above, all of the amendments made according to the provision of Article 19 (1) are made without adding new matters.
It should be noted that Claims 9 to 11 before undergoing the amendment are amended such that amended Claims 9 to 11 are limited by the structural feature of Claim 8 which was acknowledged to have an inventive step and so forth in the written opinion.

FIG. 1

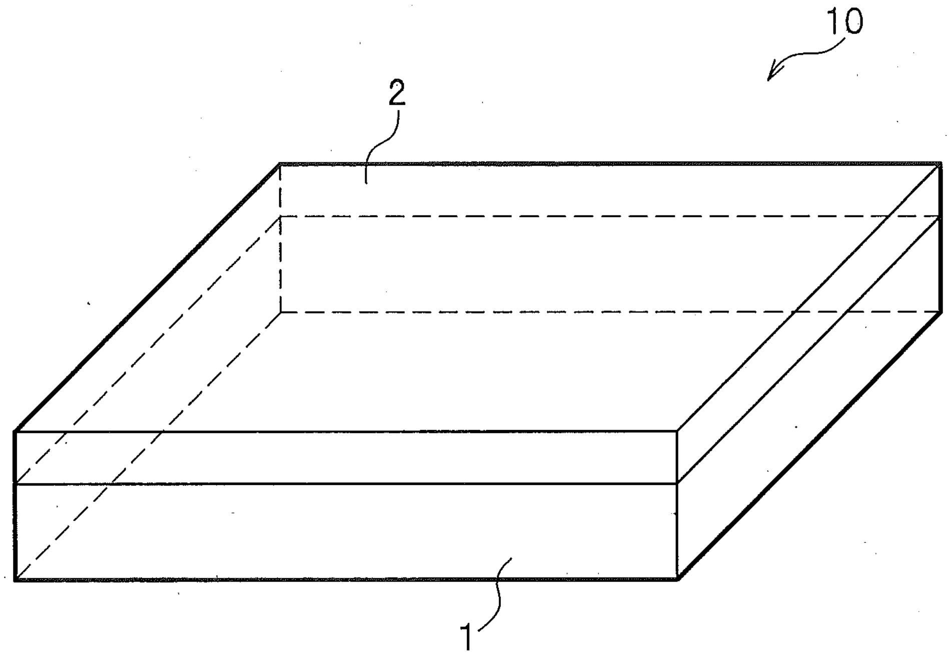

FIG.2A

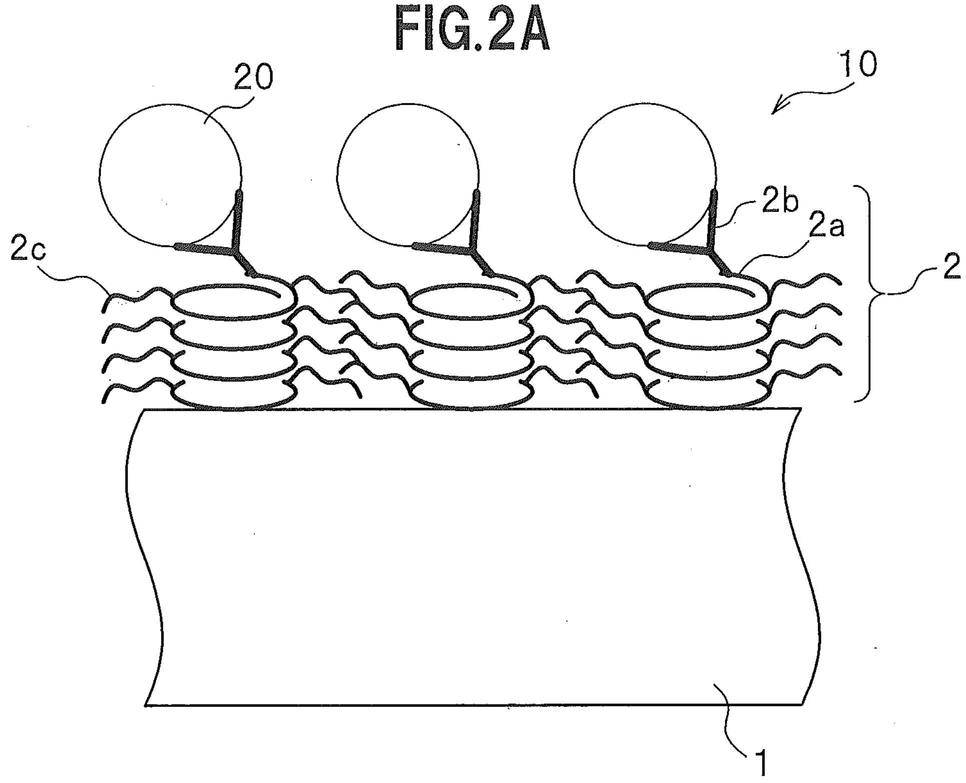


FIG.2B

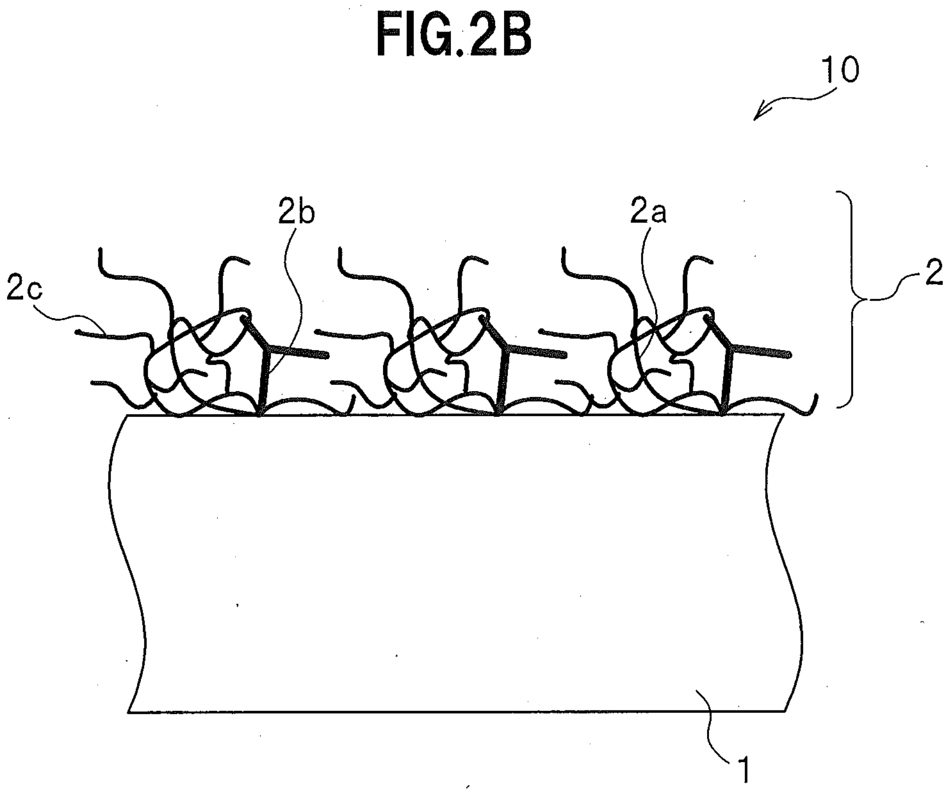

FIG.3A

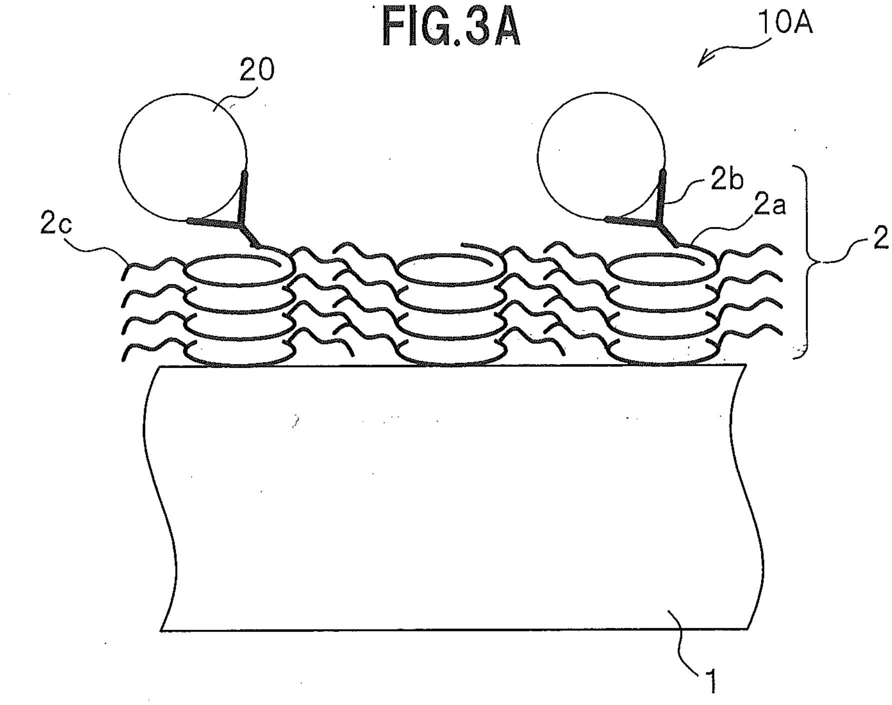


FIG.3B

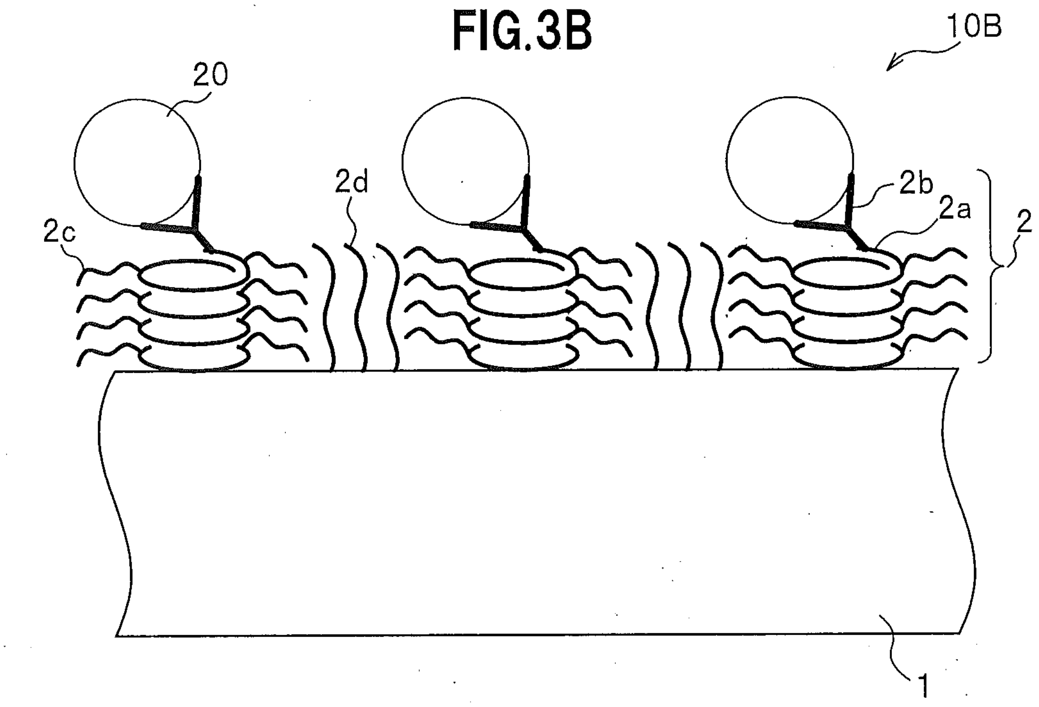

FIG.4

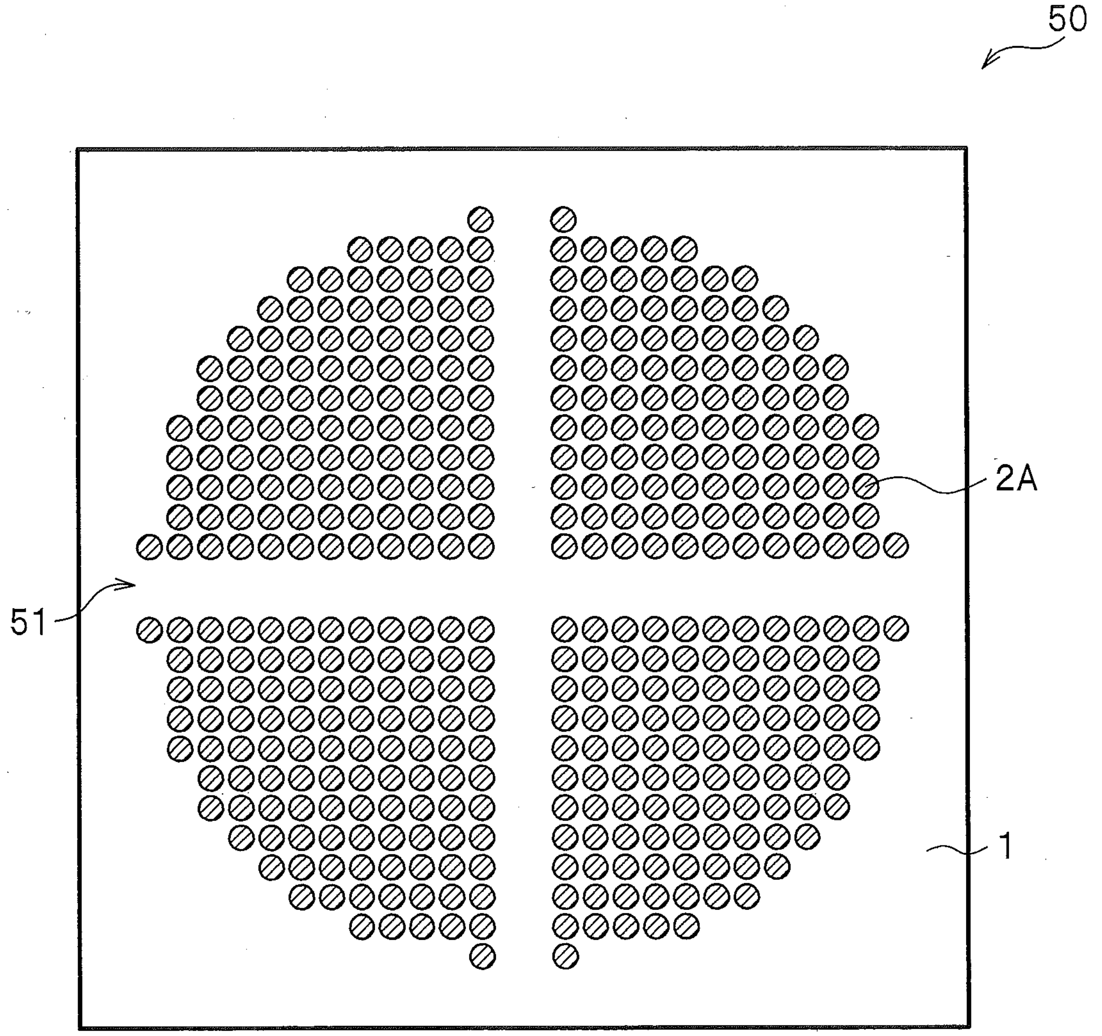

FIG.5

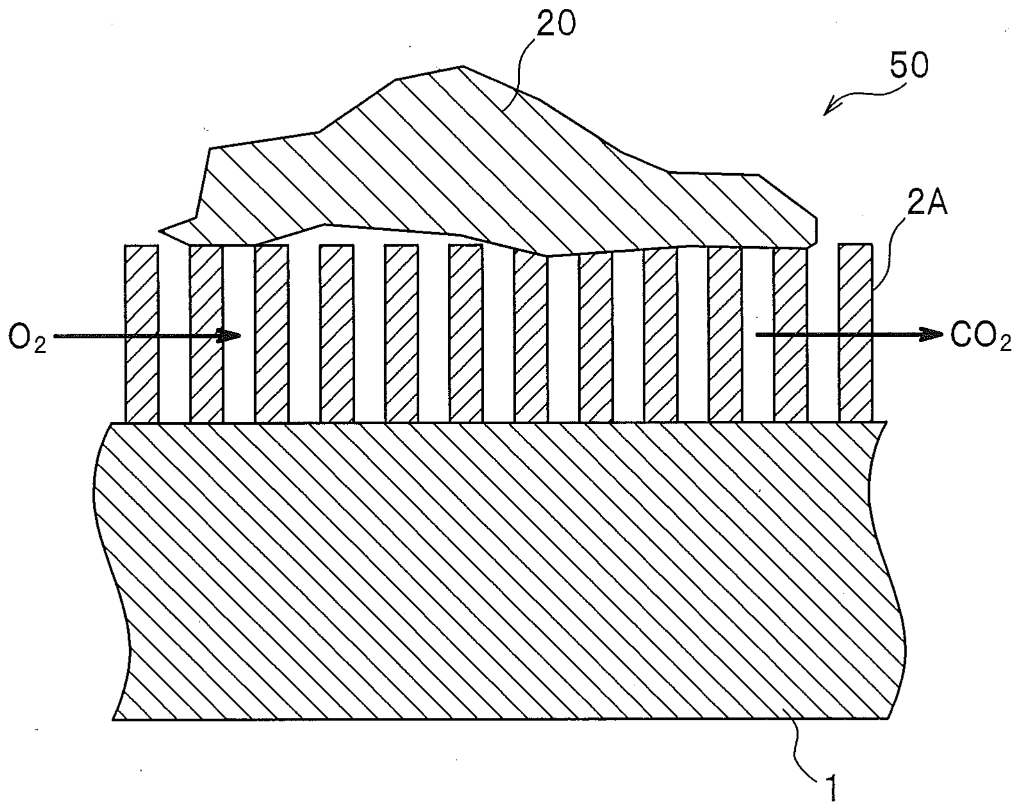

FIG.6

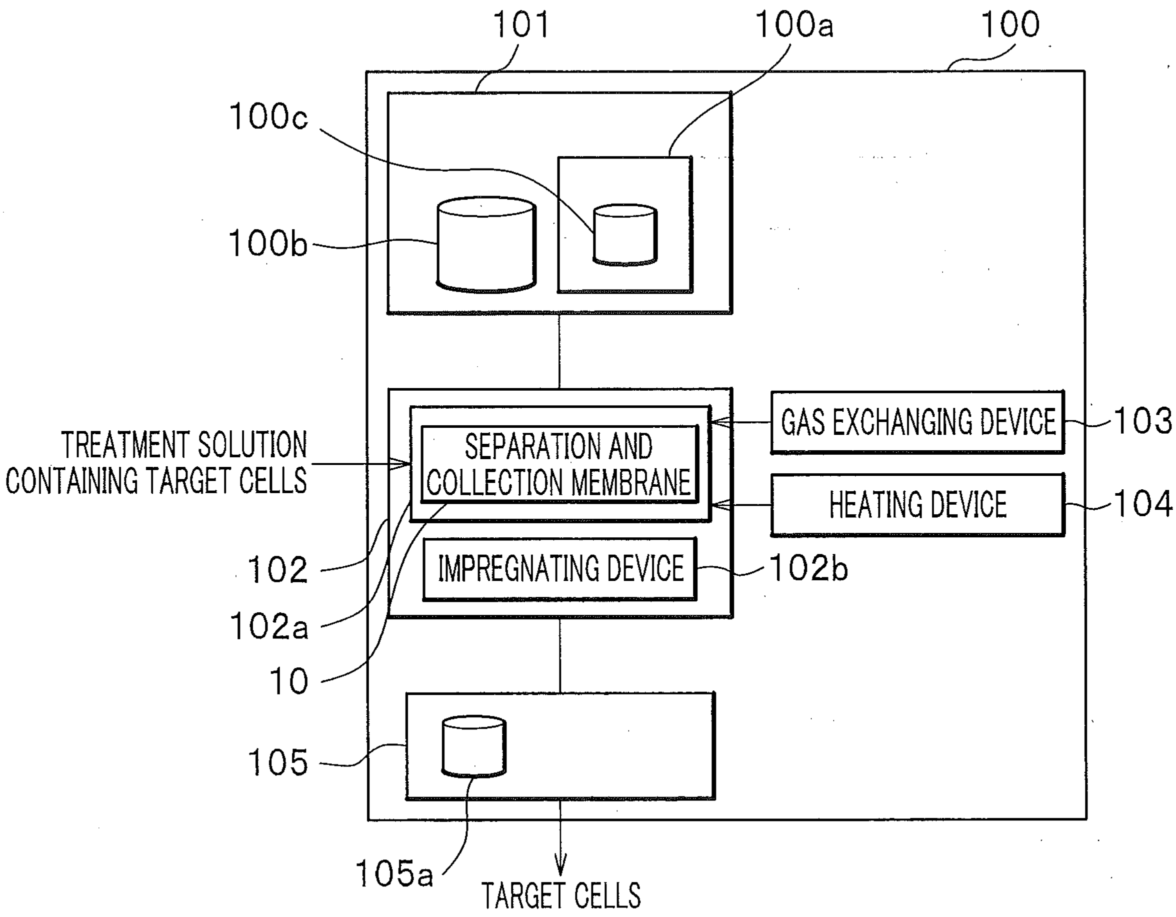
101
100a
100
100c
100b
TREATMENT SOLUTION
CONTAINING TARGET CELLS
SEPARATION AND
COLLECTION MEMBRANE
GAS EXCHANGING DEVICE
103
HEATING DEVICE
104
IMPREGNATING DEVICE
102b
102
102a
10
105
105a
TARGET CELLS

FIG.7A

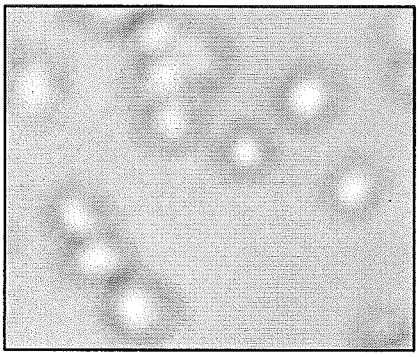

FIG.7B

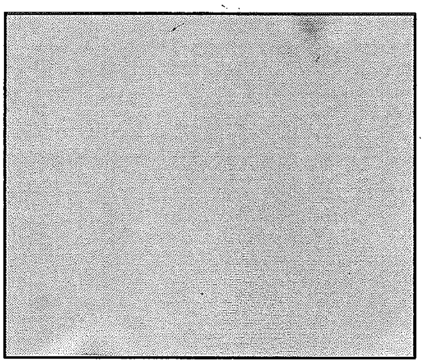

FIG.7C

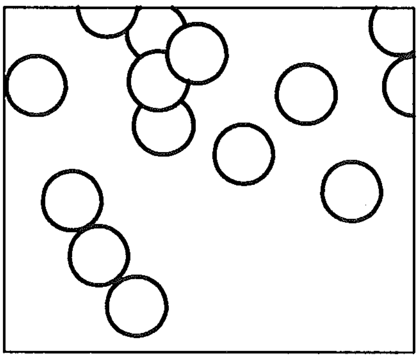

FIG.7D

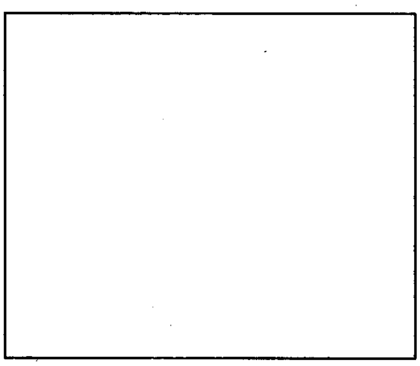

**INTERNATIONAL SEARCH REPORT**

International application No. PCT/JP2013/074186

A. CLASSIFICATION OF SUBJECT MATTER
*C12M1/00*(2006.01)i, *C12N5/071*(2010.01)i

According to International Patent Classification (IPC) or to both national classification and IPC

B. FIELDS SEARCHED

Minimum documentation searched (classification system followed by classification symbols)
C12M1/00, C12N5/071

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>A | WO 2010/027081 A1 (Tokyo University of Science),<br>11 March 2010 (11.03.2010),<br>entire text<br>& JP 2010-063375 A & JP 2010-65082 A<br>& US 2012/0015440 A1 & EP 2330182 A1 | 1-7,9,10<br>8,11 |
| X<br>A | WO 2009/072590 A1 (Tokyo University of Science),<br>11 June 2009 (11.06.2009),<br>entire text<br>(Family: none) | 1-7,9,10<br>8,11 |
| A | JP 2005-037331 A (Sumitomo Bakelite Co., Ltd.),<br>10 February 2005 (10.02.2005),<br>entire text<br>(Family: none) | 1-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 November, 2013 (29.11.13) | 10 December, 2013 (10.12.13) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2013/074186

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-046056 A (Becton, Dickinson and Co.), 22 February 2007 (22.02.2007), entire text & US 2007/0184295 A1 & EP 1750130 A1 & DE 602006009991 D & CA 2555398 A & AT 447179 T & AU 2006203325 A & CA 2555398 A1 | 1-11 |
| A | JP 2012-231788 A (Kawamura Institute of Chemical Research), 29 November 2012 (29.11.2012), entire text (Family: none) | 1-11 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012105587 A